# EUROPEAN PATENT APPLICATION

(11) **EP 4 691 411 A2**
(43) Date of publication of application: **11.02.2026**
(21) Application number: 25223581.7
(22) Date of filing: 14.06.2022
(51) Int. Cl.: A61B 90/00

(54) **JOINT IDENTIFICATION AND POSE ESTIMATION OF SURGICAL INSTRUMENTS**

(30) Priority: 16.06.2021 US 202163211099 P; 14.04.2022 US 202263330938 P
(62) Divisional of application: 22734908.1
(71) Applicant: Digital Surgery Limited, London, EC1V 2TT (GB)
(72) Inventor: ROBU, Maria Ruxandra, London (GB); SANCHEZ-MATILLA, Ricardo, London (GB); MUNTION, Imanol Luengo, London (GB); STOYANOV, Danail V., London (GB)
(74) Representative: Strange, Harry George

(57) **Abstract**

Techniques are described for improving computer-assisted surgical (CAS) systems. A CAS system includes endoscopic cameras that provide video stream of a surgical procedure. The CAS system also includes surgical instruments to perform one or more surgical actions. According to one or more aspects key points of the surgical instruments, such as tips, joints, etc., are automatically detected in the video stream. The detected key points are used to determine poses of the surgical instruments. In some aspects, detection of the instruments and estimation of poses of the respective instruments are performed concurrently.

## Description

### CROSS REFERENCE TO RELATED APPLICATIONS

This application is a PCT application that claims the benefit of U.S. Provisional Patent Application No. 63/211,099, filed on June 16, 2021. This application also claims the benefit of U.S. Provisional Patent Application No. 63/330,938, filed on April 14, 2022.

### BACKGROUND

The present disclosure is generally related to computing technology, particularly to improvements to computer-assisted surgical systems that facilitate the provision of surgical guidance based on audiovisual data and instrument data.

Computer-assisted surgery (CAS) includes the use of computer technology for surgical planning and for guiding or performing surgical interventions. CAS, in some aspects, can include or lead to robotic surgery. Robotic surgery can include a surgical instrument that performs one or more actions in relation to an action performed by medical personnel, such as a surgeon, an assistant, a nurse, etc. Alternatively, or in addition, the surgical instrument can be part of a supervisory-controlled system that executes one or more actions in a pre-programmed or pre-trained manner. Alternatively, or in addition, the medical personnel manipulates the surgical instrument in real-time. In yet other examples, the medical personnel carries out one or more actions via a platform that provides controlled manipulations of the surgical instrument based on the personnel's actions.

Computer-assisted systems can be useful to augment a person's physical sensing, perception, and reaction capabilities. For example, such systems can effectively provide the information corresponding to an expanded field of vision, both temporal and spatial, enabling a person to adjust current and future actions based on the information. Highly variable, dynamic, and/or unpredictable environments present challenges in defining rules that indicate how representations of the environments are to be processed to output data to productively assist the person in action performance. Further, identifying and tracking multiple objects in complex scenes can be challenging where variations in lighting, obstructions, and orientation of the objects may occur.

### BRIEF DESCRIPTION

According to one or more aspects, a system includes a memory device and one or more processors coupled with the memory device. One or more processors are configured to perform the method(s) described herein.

According to one or more aspects, a computer program product includes a memory device with computer-readable instructions stored thereon, wherein executing the computer-readable instructions by one or more processing units causes the one or more processing units to perform the method(s) described herein.

According to one or more aspects, a computer-implemented method is described to estimate, autonomously using machine learning, a pose of a surgical instrument in a video of a surgical procedure. The pose is estimated by identifying, using machine learning, one or more key points of the surgical instrument. The key points can include joints, tips, shaft endpoints, etc.

A system includes a memory device and one or more processors coupled with the memory device. The one or more processors identify, autonomously, one or more key points associated with a plurality of surgical instruments in a video of a surgical procedure. The one or more processors group the one or more key points according to the plurality of surgical instruments using a first machine learning model. The one or more processors determine, based on the one or more key points that are grouped, poses of the surgical instruments and types of the surgical instruments, respectively, using a second machine learning model, wherein the poses and the types are determined concurrently.

In one or more aspects, a pose of a surgical instrument from the plurality of surgical instruments is used to provide user feedback.

In one or more aspects, the one or more processors are further configured to generate a bounding box of a surgical instrument based on the one or more key points grouped according to the surgical instrument.

In one or more aspects, the video of the surgical procedure is captured by an endoscopic camera from inside a patient's body.

In one or more aspects, the video of the surgical procedure is captured by a camera from outside a patient's body.

In one or more aspects, the first machine learning model outputs an annotation for each of the one or more key points identified.

In one or more aspects, the poses and types of the surgical instruments are identified with temporal continuity.

In one or more aspects, the one or more processors are configured to test a surgical robotic arm, by issuing a command to the surgical robotic arm that results in a surgical instrument associated with the surgical robotic arm to be in a predetermined pose. Further, the testing includes determining a first pose of the surgical instrument based on the one or more key points that are grouped. Further, the testing includes comparing the first pose and the predetermined pose.

According to one or more aspects, a computer-implemented method includes identifying, autonomously, one or more key points associated with a plurality of surgical instruments in a video of a surgical procedure. Further, the method includes grouping a set of key points from the one or more key points associated with a surgical instrument using a first machine learning model. Further, the method includes determining, based on the set of key points that are grouped, a pose of the surgical instrument.

In one or more aspects, the method further includes depicting a graphical overlay on the video to indicate the identified pose of the surgical instrument.

In one or more aspects, the graphical overlay includes a depiction of the one or more key points to identify an exit path to move the surgical instrument.

In one or more aspects, the method further includes, in response to the pose of the surgical instrument matching a threshold pose, generating a user notification.

In one or more aspects, the user notification is a first user notification, and in response to the pose of the surgical instrument not matching the threshold pose, generating a second user notification, different from the first user notification.

In one or more aspects, the threshold pose is indicative of a desired pose of the surgical instrument based on a surgical action to be performed.

In one or more aspects, the threshold pose is indicative of an undesired pose of the surgical instrument.

In one or more aspects, the user notification includes an audible notification.

In one or more aspects, the user notification is provided on a separate display, distinct from the video.

According to one or more aspects, a computer program product includes a memory device with computer-readable instructions stored thereon, wherein executing the computer-readable instructions by one or more processing units causes the one or more processing units to perform a method. The method includes accessing a video of a surgical procedure comprising use of a plurality of surgical instruments concurrently. Further, the method includes identifying, autonomously, one or more key points associated with the surgical instruments. Further, the method includes concurrently performing, using one or more machine learning models, grouping a set of key points from the one or more key points, the set of key points associated with a surgical instrument; identifying a type of the surgical instrument based on the set of key points, and estimating a pose of the surgical instrument based on the set of key points. Further, the method includes augmenting the video of the surgical procedure in response to a key point of the surgical instrument being out of view from the video.

In one or more aspects, the one or more machine learning models include multi-tasking convolutional neural network layers that aggregate spatio-temporal features in one or more frames of the video.

In one or more aspects, the method further includes augmenting the video of the surgical procedure in response to the key point of the surgical instrument being within a predetermined proximity of an anatomical structure.

Additional technical features and benefits are realized through the techniques of the present invention. Aspects of the invention are described in detail herein and are considered a part of the claimed subject matter. For a better understanding, refer to the detailed description and to the drawings.

### BRIEF DESCRIPTION OF THE DRAWINGS

The specifics of the exclusive rights described herein are particularly pointed out and distinctly claimed in the claims at the conclusion of the specification. The foregoing and other features and advantages of the aspects of the invention are apparent from the following detailed description taken in conjunction with the accompanying drawings in which:
FIG. 1 depicts an example computer-assisted surgery system according to one or more aspects;
FIG. 2 shows a system for identifying surgical instruments and estimating poses of surgical instruments in surgical data using machine learning according to one or more aspects;
FIG. 3 depicts example surgical instruments with key points identified and annotated with labels according to one or more aspects;
FIG. 4 depicts a variability in the annotations when the same frame is annotated by multiple different annotators according to one or more aspects;
FIG. 5 depicts an example of using a machine learning model to localize and group the annotation variability according to one or more aspects;
FIG. 6 depicts the example of training data used to train the machine learning model according to one or more aspects;
FIG. 7 depicts a flowchart of a method for detection of key points and poses of surgical instruments in surgical data using machine learning according to one or more aspects;
FIG. 8 depicts an example result of surgical instrument detection using one or more aspects herein.
FIG. 9 depicts a machine learning model (neural network) architecture according to one or more aspects for performing joint detection, identification, and pose estimation of surgical instruments;
FIG. 10 depicts a surgical procedure system in accordance with one or more aspects;
FIG. 11 depicts an example computing system that can be used to implement one or more aspects described herein.

The diagrams depicted herein are illustrative. There can be many variations to the diagram or the operations described therein without departing from the spirit of the invention. For instance, the actions can be performed in a differing order, or actions can be added, deleted, or modified. Also, the term "coupled" and variations thereof describe having a communications path between two elements and do not imply a direct connection between the elements with no intervening elements/connections between them. All of these variations are considered a part of the specification.

### DETAILED DESCRIPTION

Exemplary aspects of technical solutions described herein relate to, among other things, devices, systems, methods, computer-readable media, techniques, and methodologies for using machine learning and computer vision to improve computer-assisted surgical systems. In one or more aspects, structures, such as anatomical structures, surgical instruments, and other artifacts, are predicted dynamically and substantially in real-time as the surgical data is being captured and analyzed by technical solutions described herein. Exemplary aspects of technical solutions described herein further facilitate generating augmented views of surgical sites using semantic surgical representations based on the predictions of one or more structures in the surgical data.

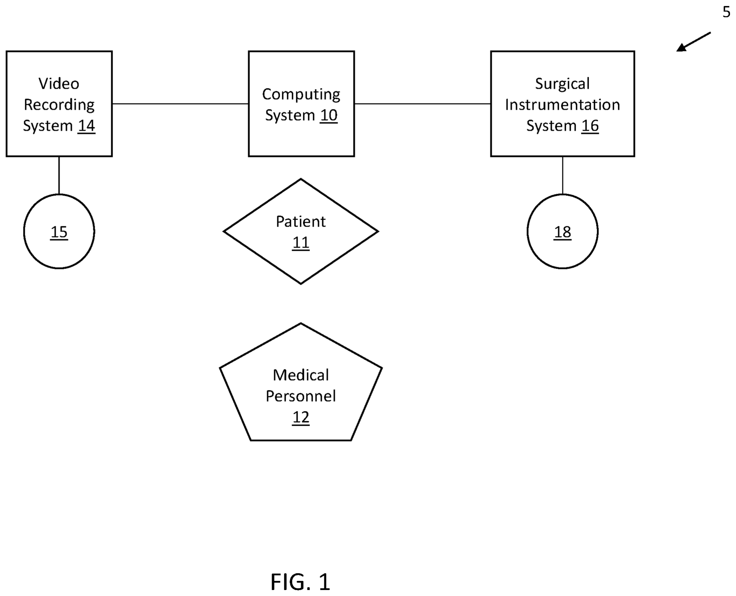

FIG. 1 depicts an example CAS system according to one or more aspects. The CAS system 100 includes at least a computing system 10, a video recording system 14, and a surgical instrumentation system 16.

Actor 12 can be medical personnel that uses the CAS system 100 to perform a surgical procedure on a patient 11. Medical personnel can be a surgeon, assistant, nurse, administrator, or any other actor that interacts with the CAS system 100 in a surgical environment. The surgical procedure can be any type of surgery, such as but not limited to cataract surgery, laparoscopic cholecystectomy, endoscopic endonasal transsphenoidal approach (eTSA) to resection of pituitary adenomas, or any other surgical procedure. In other examples, actor 12 can be a technician, an administrator, an engineer, or any other such personnel that interacts with the CAS system 100. For example, actor 12 can record data from the CAS system 100, configure/update one or more attributes of the CAS system 100, review past performance of the CAS system 100, repair the CAS system 100, etc.

A surgical procedure can include multiple phases, and each phase can include one or more surgical actions. A "surgical action" can include an incision, a compression, a stapling, a clipping, a suturing, a cauterization, a sealing, or any other such actions performed to complete a phase in the surgical procedure. A "phase" represents a surgical event that is composed of a series of steps (e.g., closure). A "step" refers to the completion of a named surgical objective (e.g., hemostasis). During each step, certain surgical instruments 18 (e.g., forceps) are used to achieve a specific objective by performing one or more surgical actions.

The surgical instrumentation system 16 provides electrical energy to operate one or more surgical instruments 18 to perform the surgical actions. The electrical energy triggers an activation in the surgical instrument 18. The electrical energy can be provided in the form of an electrical current or an electrical voltage. The activation can cause a surgical action to be performed. The surgical instrumentation system 16 can further include electrical energy sensors, electrical impedance sensors, force sensors, bubble and occlusion sensors, and various other types of sensors. The electrical energy sensors can measure and indicate an amount of electrical energy applied to one or more surgical instruments 18 being used for the surgical procedure. The impedance sensors can indicate an amount of impedance measured by the surgical instruments 18, for example, from the tissue being operated upon. The force sensors can indicate an amount of force being applied by the surgical instruments 18. Measurements from various other sensors, such as position sensors, pressure sensors, and flow meters, can also be input.

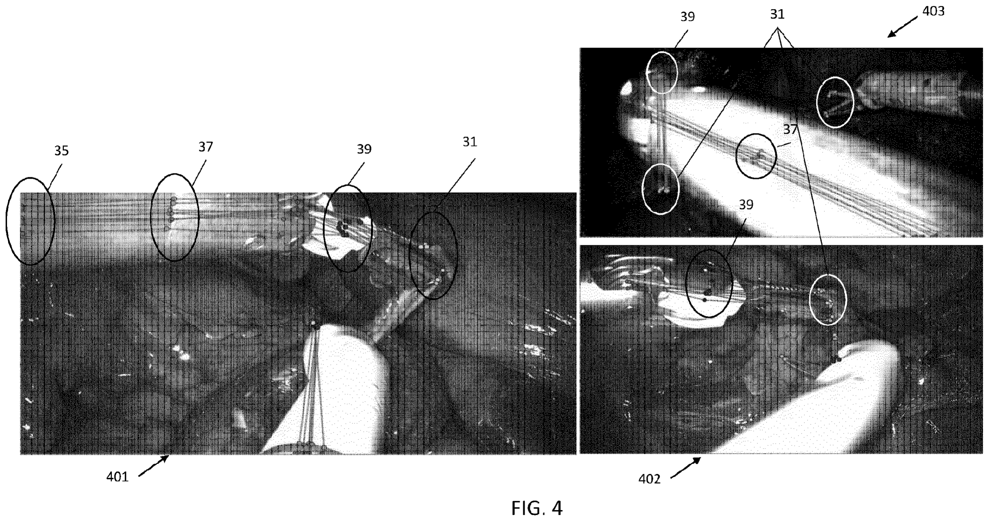

The video recording system 14 includes one or more cameras 15, such as operating room cameras, endoscopic cameras, etc. The cameras capture video data of the surgical procedure being performed. The video recording system 14 includes one or more video capture devices 15 that can include cameras 15 placed in the surgical room to capture events surrounding (i.e., outside) the patient being operated upon. The video recording system 14 further includes cameras 15 that are passed inside (e.g., endoscopic cameras) the patient to capture endoscopic data. The endoscopic data provides video images of the surgical procedure (e.g., FIG. 4).

The computing system 10 includes one or more memory devices, one or more processors, and a user interface device, among other components. The computing system 10 can execute one or more computer-executable instructions. The execution of the instructions facilitates the computing system 10 to perform one or more methods, including those described herein. The computing system 10 can communicate with other computing systems via a wired and/or a wireless network. In one or more examples, the computing system 10 includes one or more trained machine learning models that can detect and/or predict features of/from the surgical procedure that is being performed or has been performed earlier. Features can include structures such as anatomical structures and surgical instruments (18) in the surgical procedure. Features can further include events such as phases and actions in the surgical procedure. Features that are detected can further include actor 12 and patient 11. Based on the detection, the computing system 10, in one or more examples, can provide recommendations for subsequent actions to be taken by actor 12. Alternatively, or in addition, the computing system 10 can provide one or more reports based on the detections. The detections by the machine learning models can be performed in an autonomous or semi-autonomous manner.

The machine learning models can include artificial neural networks, such as deep neural networks, convolutional neural networks, recurrent neural networks, encoders, decoders, or any other type of machine learning models. The machine learning models can be trained in a supervised, unsupervised, or hybrid manner. The machine learning models can be trained to perform detection and/or prediction using one or more types of data acquired by the CAS system 100. For example, the machine learning models can use the video data captured via the video recording system 14. Alternatively, or in addition, the machine learning models use the surgical instrumentation data from the surgical instrumentation system 16. In yet other examples, the machine learning models use a combination of the video and the surgical instrumentation data.

Additionally, in some examples, the machine learning models can also use audio data captured during the surgical procedure. The audio data can include sounds emitted by the surgical instrumentation system 16 while activating one or more surgical instruments 18. Alternatively, or in addition, the audio data can include voice commands, snippets, or dialog from one or more actors 12. The audio data can further include sounds made by the surgical instruments 18 during their use.

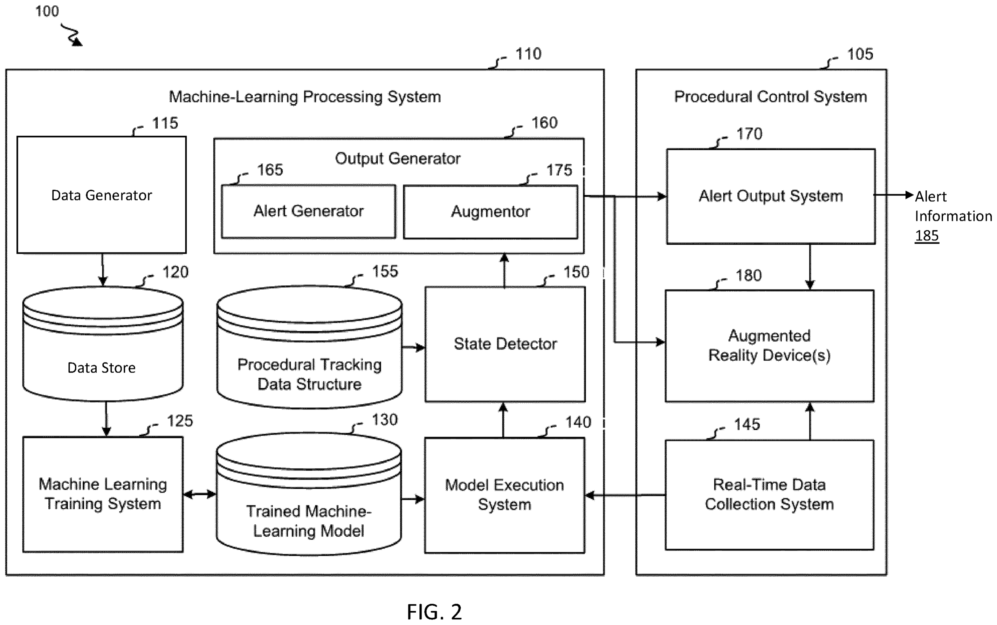

FIG. 2 shows a system 100 for identifying surgical instruments and estimating poses of surgical instruments in surgical data using machine learning according to one or more aspects. System 100 uses data streams that are part of the surgical data to identify procedural states and surgical instruments according to some aspects. System 100 includes a procedural control system 105 that collects image data and coordinates outputs responsive to predicted structures and states. The procedural control system 105 can include one or more devices (e.g., one or more user devices and/or servers) located within and/or associated with a surgical operating room and/or control center. System 100 further includes a machine-learning processing system 110 that processes the surgical data using one or more machine-learning models to identify one or more features from the input data and further estimate one or more aspects of the features. It will be appreciated that machine-learning processing system 110 can include one or more devices (e.g., one or more servers), each of which can be configured to include part or all of one or more of the depicted components of the machine-learning processing system 110. In some instances, a part or all of machine-learning processing system 110 is in the cloud and/or remote from an operating room and/or physical location corresponding to a part or all of procedural control system 105. For example, the machine-learning training system 125 can be a separate device (e.g., server) that stores its output as the one or more trained machine-learning models 130, which are accessible by the model execution system 140, separate from the machine-learning training system 125. In other words, in some aspects, devices that "train" the models are separate from devices that "infer," i.e., perform real-time processing of surgical data using the trained models 130.

Machine-learning processing system 110 includes a data generator 115 configured to generate simulated surgical data, such as a set of virtual images, or record surgical data from ongoing procedures, to train one or more machine-learning models. Data generator 115 can access (read/write) a data store 120 with recorded data, including multiple images and/or multiple videos. The images and/or videos can include images and/or videos collected during one or more procedures (e.g., one or more surgical procedures). For example, the images and/or video may have been collected by a user device worn by a participant (e.g., surgeon, surgical nurse, anesthesiologist, etc.) during the surgery and/or by a non-wearable imaging device located within an operating room.

Each of the images and/or videos included in the recorded data can be defined as a base image and can be associated with other data that characterizes an associated procedure and/or rendering specifications. For example, the other data can identify a type of procedure, a location of a procedure, one or more people involved in performing the procedure, surgical objectives, and/or an outcome of the procedure. Alternatively, or in addition, the other data can indicate a stage of the procedure with which the image or video corresponds, rendering specification with which the image or video corresponds, and/or a type of imaging device that captured the image or video (e.g., and/or, if the device is a wearable device, a role of a particular person wearing the device, etc.). Further, the other data can include image-segmentation data that identifies and/or characterizes one or more objects (e.g., tools, anatomical objects, etc.) that are depicted in the image or video. The characterization can indicate the position, orientation, or pose of the object in the image. For example, the characterization can indicate a set of pixels that correspond to the object and/or a state of the object resulting from a past or current user handling. Localization can be performed using a variety of techniques for identifying objects in one or more coordinate systems.

Data generator 115 identifies one or more sets of rendering specifications for the set of virtual images. An identification is made as to which rendering specifications are to be specifically fixed and/or varied. Alternatively, or in addition, the rendering specifications that are to be fixed (or varied) are predefined. The identification can be made based on, for example, input from a client device, a distribution of one or more rendering specifications across the base images and/or videos, and/or a distribution of one or more rendering specifications across other image data. For example, if a particular specification is substantially constant across a sizable data set, the data generator 115 defines a fixed corresponding value for the specification. As another example, if rendering-specification values from at least a predetermined amount of data span across a range, the data generator 115 defines the rendering specifications based on the range (e.g., to span the range or to span another range that is mathematically related to the range of distribution of the values).

A set of rendering specifications can be defined to include discrete or continuous (finely quantized) values. A set of rendering specifications can be defined by a distribution, such that specific values are to be selected by sampling from the distribution using random or biased processes.

One or more sets of rendering specifications can be defined independently or in a relational manner. For example, if the data generator 115 identifies five values for a first rendering specification and four values for a second rendering specification, the one or more sets of rendering specifications can be defined to include twenty combinations of the rendering specifications or fewer (e.g., if one of the second rendering specifications is only to be used in combination with an incomplete subset of the first rendering specification values or the converse). In some instances, different rendering specifications can be identified for different procedural states and/or other metadata parameters (e.g., procedural types, procedural locations, etc.).

Using the rendering specifications and base image data, the data generator 115 generates simulated surgical data (e.g., a set of virtual images), which is stored at the data store 120. For example, a three-dimensional model of an environment and/or one or more objects can be generated using the base image data. Virtual image data can be generated using the model to determine - given a set of particular rendering specifications (e.g., background lighting intensity, perspective, zoom, etc.) and other procedure-associated metadata (e.g., a type of procedure, a procedural state, a type of imaging device, etc.). The generation can include, for example, performing one or more transformations, translations, and/or zoom operations. The generation can further include adjusting the overall intensity of pixel values and/or transforming RGB (e.g., red/green/blue) values to achieve particular color-specific specifications.

A machine-learning training system 125 uses the recorded data in the data store 120, which can include the simulated surgical data (e.g., a set of virtual images) and actual surgical data to train one or more machine-learning models. The machine-learning models can be defined based on a type of model and a set of hyperparameters (e.g., defined based on input from a client device). The machine-learning models can be configured based on a set of parameters that can be dynamically defined based on (e.g., continuous or repeated) training (i.e., learning, parameter tuning). Machine-learning training system 125 can use one or more optimization algorithms to define the set of parameters to minimize or maximize one or more loss functions. The set of (learned) parameters can be stored as a trained machine-learning model data structure 130, which can also include one or more non-learnable variables (e.g., hyperparameters and/or model definitions).

A model execution system 140 can access the machine-learning model data structure 130 and accordingly configure one or more machine-learning models for inference (i.e., prediction). One or more machine-learning models can include, for example, a fully convolutional network adaptation, an adversarial network model, or other types of models, as indicated in data structure 130. One or more machine-learning models can be configured in accordance with one or more hyperparameters and the set of learned parameters.

The one or more machine-learning models, during execution, can receive, as input, surgical data to be processed and generate one or more inferences according to the training. For example, the surgical data can include data streams (e.g., an array of intensity, depth, and/or RGB values) for a single image or for each of a set of frames (e.g., including multiple images or an image with sequencing data) representing a temporal window of fixed or variable length in a video. The surgical data that is input can be received from a real-time data collection system 145, which can include one or more devices located within an operating room and/or streaming live imaging data collected during the performance of a procedure. Video processing can include decoding and/or decompression when a video stream is received in an encoded or compressed format such that data for a sequence of images can be extracted and processed. The surgical data can include additional data streams, such as audio data, RFID data, textual data, measurements from one or more surgical instruments/sensors, etc., that can represent stimuli/procedural states from the operating room. The different inputs from different devices/sensors are synchronized before inputting into the model.

One or more machine-learning models can analyze the surgical data and, in one or more aspects, predict and/or estimate and/or characterize structures included in the visual data from the surgical data. The visual data can include image and/or video data in the surgical data. The prediction and/or characterization of the structures can include segmenting the visual data or predicting the localization of the structures with a probabilistic heatmap. In some instances, the one or more machine-learning models include or are associated with a preprocessing or augmentation (e.g., intensity normalization, resizing, cropping, etc.) that is performed prior to segmenting the visual data. An output of the one or more machine-learning models can include image-segmentation or probabilistic heatmap data that indicates which (if any) of a defined set of structures are predicted within the visual data, a location and/or position and/or pose of the structure(s) within the image data, and/or state of the structure(s). The location can be a set of coordinates in the image data. For example, the coordinates can provide a bounding box, one or more key points, graphical overlays, or any other such visual cues about the structures. The coordinates can provide boundaries that surround the structure(s) being predicted. One or more machine-learning models can be trained to perform higher-level predictions and tracking, such as predicting a state of a surgical procedure and tracking one or more surgical instruments used in the surgical procedure, as further described herein.

A state detector 150 can use the output from the execution of the machine-learning model to identify a state within a surgical procedure ("procedure"). A procedural tracking data structure can identify a set of potential states that can correspond to part of a performance of a specific type of procedure. Different procedural data structures (e.g., different machine-learning-model parameters and/or hyperparameters) may be associated with different types of procedures. The data structure can include a set of nodes, with each node corresponding to a potential state. The data structure can include directional connections between nodes that indicate (via the direction) an expected order during which the states will be encountered throughout an iteration of the procedure. The data structure may include one or more branching nodes that feed to multiple next nodes and/or can include one or more points of divergence and/or convergence between the nodes. In some instances, a procedural state indicates a procedural action (e.g., surgical action) that is being performed or has been performed and/or indicates a combination of actions that have been performed. In some instances, a procedural state relates to a biological state of a patient undergoing a surgical procedure. For example, the biological state can indicate a complication (e.g., blood clots, clogged arteries/veins, etc.), precondition (e.g., lesions, polyps, etc.).

Each node within the data structure can identify one or more characteristics of the state. The characteristics can include visual characteristics. In some instances, the node identifies one or more tools that are typically in use or availed for use (e.g., on a tool tray) during the state, one or more roles of people who are typically performing a surgical task, a typical type of movement (e.g., of a hand or tool), etc. Thus, state detector 150 can use the segmented data generated by model execution system 140 that indicates the presence and/or characteristics of particular objects within a field of view to identify an estimated node to which the real image data corresponds. Identification of the node (and/or state) can further be based upon previously detected states for a given procedural iteration and/or other detected input (e.g., verbal audio data that includes person-to-person requests or comments, explicit identifications of a current or past state, information requests, etc.).

An output generator 160 can use the state to generate an output. Output generator 160 can include an alert generator 165 that generates and/or retrieves information associated with the state and/or potential next events. For example, the information can include details as to warnings and/or advice corresponding to current or anticipated procedural actions. The alert generator 165 can be configured to communicate with one or more other systems, such as procedural control system 105, to provide notice or trigger actions based on the information. The information can further include one or more events for which to monitor. The information can identify the next recommended action. The alert generator 165 can deliver tips and alerts to a surgical team to improve team coordination based on the state of the procedure. Machine learning can be used to determine the remaining time of an operation to help with the preparation and scheduling of the facilities for subsequent use. Alerts can be generated to warn or notify surgeons or other parties through various devices and systems.

The user feedback can be transmitted to an alert output system 170, which can cause the user feedback to be output via a user device and/or other devices that is (for example) located within the operating room or control center. The user feedback can include a visual, audio, tactile, or haptic output that is indicative of the information. The user feedback can facilitate alerting an operator, for example, a surgeon or any other user of the system 100. The alert output system 170 may also provide alert information 185 to one or more other systems (not depicted).

Output generator 160 can also include an augmenter 175 that generates or retrieves one or more graphics and/or text to be visually presented on (e.g., overlaid on) or near (e.g., presented underneath or adjacent to or on a separate screen) in real-time capture of a procedure. Augmenter 175 can further identify where the graphics and/or text are to be presented (e.g., within a specified size of a display). In some instances, a defined part of a field of view is designated as being a display portion to include augmented data. In some instances, the position of the graphics and/or text is defined so as not to obscure the view of an important part of an environment for the surgery and/or to overlay particular graphics (e.g., of a tool) with the corresponding real-world representation.

Augmenter 175 can send the graphics and/or text and/or any positioning information to an augmented reality device 180, which can integrate the graphics and/or text with a user's environment in real-time as an augmented reality visualization. Augmented reality device 180 can include a pair of goggles that can be worn by a person participating in part of the procedure. It will be appreciated that, in some instances, the augmented display can be presented on a non-wearable user device, such as a computer or tablet. The augmented reality device 180 can present the graphics and/or text at a position as identified by augmenter 175 and/or at a predefined position. Thus, a user can maintain a real-time view of procedural operations and further view pertinent state-related information.

In one or more examples, the machine learning models can detect surgical actions, surgical phases, anatomical structures, surgical instruments, and various other features from the data associated with a surgical procedure. The detection can be performed in real-time in some examples. Alternatively, or in addition, the computing system 10 analyzes the surgical data, i.e., the various types of data captured during the surgical procedure, in an offline manner (e.g., post-surgery).

A technical challenge with using CAS system 100 is to determine how the surgical instruments 18 interact with anatomy. For example, when performing surgical actions such as grasping, cutting, etc., the tips of surgical instruments 18 may be kept open/closed, leading to different outcomes. Identifying and further recording the particular option used during particular surgical procedures or surgical actions can be beneficial. For example, such information can be used when a similar case arises in the future. Additionally, it can be beneficial to identify and keep a record of a count of specific instrument parts (e.g., needle, swab) during the surgical procedure. Such information can be used for detailed post-operative analysis, for example, accounting for all of the instrument parts being extracted from the patient 11. Further yet, detecting and recording trajectories and motion of the surgical instruments 18 over time during the surgical procedure is also a technical challenge. Such information can also be used in real-time, for example, to prevent potential damage to tissue with early-warning systems.

Technical solutions described herein address such technical challenges by detecting real-time granular information of the positions and orientations of the surgical instrument 18. The pose (position and orientation) of the surgical instrument 18 can be detected using machine learning in one or more examples.

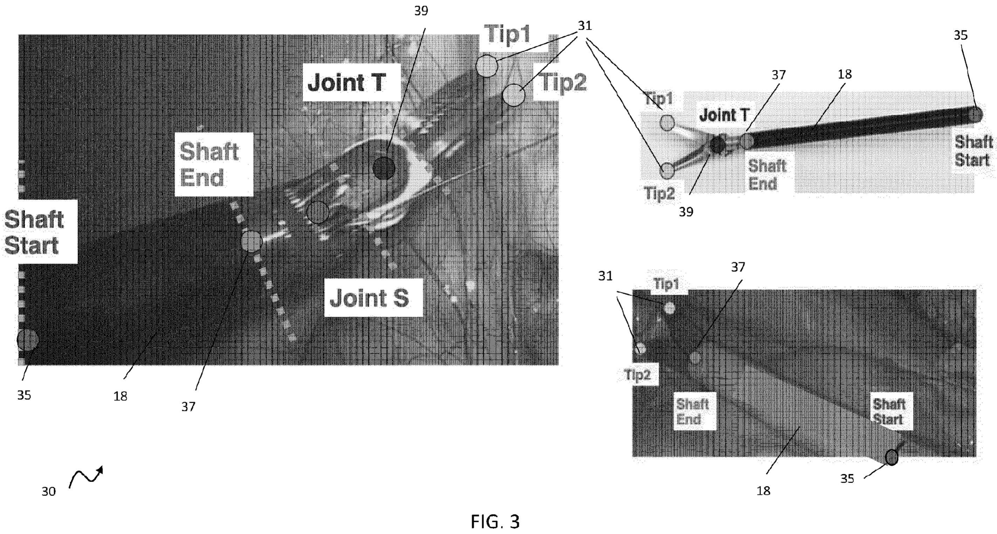

FIG. 3 depicts example surgical instruments 18 with key points identified and annotated with labels. It is understood that different surgical instruments can have different key points 30 and corresponding labels. In the depicted example, the key points 30 of the surgical instruments 18 include shaft-start 35 and shaft-end 37, one or more joints 39 that provide one or more degrees of freedom to move the instrument 18, and one or more tips 31 that is used to perform a surgical action. The annotated data shown is one example, and in other aspects, the annotation can be performed in another manner. The annotated data is used to train a machine learning model to identify and localize the key points 30 in video data that do not include annotations. The localization facilitates identifying key points 30 belonging to different surgical instruments 18 and grouping the key points 30 based on the instrument 18 they belong to.

Annotating the key points of the surgical instruments in a surgical video (e.g., an endoscopic video or a video recorded by a camera external to the patient's body) can be a technical challenge in itself. The annotation can vary from one operator to another.

FIG. 4 depicts a variability in the annotations when the same frame is annotated by multiple different operators, in this particular case, 10 different annotators. Annotation precision depends on the scale of instruments, key point type, occlusions, and pose of the instrument 18, among other factors. Accordingly, as can be seen, the different annotators exhibit such a variation by identifying and labeling different locations (i.e., pixels or coordinates) in the images 401, 402, and 403, as the shaft-starts 35, shaft-ends 37, joints 39, and tips 31.

Technical solutions described herein address such a technical challenge of annotation variability by localization and grouping of the predictions. Such localization and grouping are performed using a machine learning model(s).

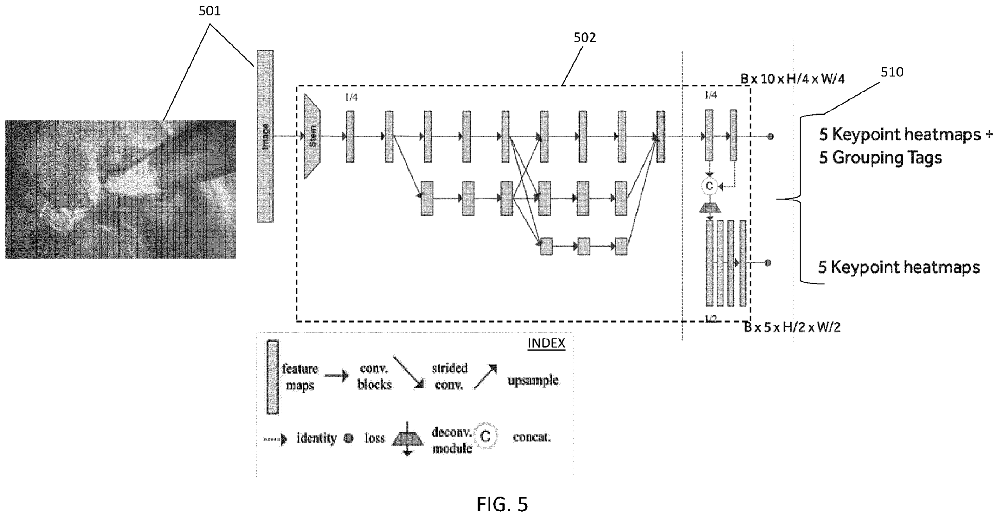

FIG. 5 depicts an example of using a machine learning model to localize and group the annotation variability according to one or more examples. The example shown in FIG. 5 requires one machine learning model to identify instrument types and another machine learning model that will detect/localize the instrument. In other aspects, a single machine learning model performs both 1) key point detection+localization and 2) instrument identification based on the localization.

An input data 501 is passed to the machine learning model 502, which outputs feature maps 510. The input data 501 can have dimensions represented as [B, H, W], where B is a batch size (number of images used in a single pass of the model); H is an image height, and W is image width. Different values of B, H, and W can be used by one or more aspects of the technical solutions described herein. The outputs of one or more branches of the machine learning models can have the same or different dimensions than the input data 501. For example, the top branch of the model can output features with dimensions [B, 10, H/4, W/4], i.e., the image size is reduced compared to the input data 501. The batch size can also vary. It is understood that the above is one example and that other variations are possible in other aspects of the technical solutions herein. For example, a bottom branch of the model outputs features with dimensions [B, 5 H/2, W/2]. In this particular example, the top-branch outputs smaller spatial resolution and larger channel number, whereas the bottom-branch outputs higher spatial resolution but lower channel number.

In the feature maps 510, the annotation variability is localized and grouped so that the feature maps 510 can provide instrument identification and their poses in real-time (unseen) video data. Alternatively, in some aspects, the feature maps 510 that are output have to be further analyzed by training another (second) machine learning model to detect and identify instruments 18 and their poses in real-time (unseen) video data. For example, the real-time video data that is processed to detect the key points in the surgical instruments 18 can include streaming data as the surgical procedure is being performed. Alternatively, or in addition, the video data can be processed offline during post-operative analysis. Accordingly, the localization and grouping machine learning model (502) improves the (second) machine learning model that autonomously detects poses of the instruments 18 in the input data 501. Without localization and grouping, the (second) machine learning model can output only output a rough estimate of the instrument 18, without any precise information about where the joints/tips (key points 30) are, for example, a rectangle around each instrument 18.

Alternatively, or in addition, the localization and grouping also facilitate determining key points 30 associated with the same instruments and providing only the key points of the same instrument for identifying the instrument 18 and the instrument's pose.

The machine learning model 502, in one or more aspects, includes one or more encoders, one or more convolutional layers, and one or more deconvolutional layers, among other types of computational layers. It is understood that in other aspects, the structure of the machine learning model 502 can be different from what is shown in FIG. 5.

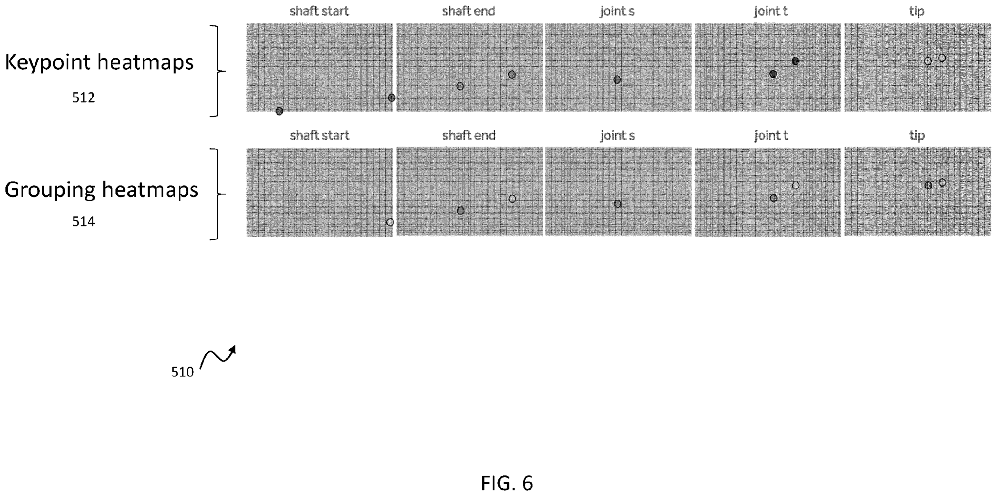

FIG. 6 depicts the example of training data used to train the machine learning model 502 according to one or more aspects. Depicted examples illustrate how keypoint annotations are transformed into labels that can be used to train the model 502. The labels teach the model 502, where the key points 30 are in each image and their grouping within one instrument. The output 510 of the machine learning model 502 includes a plurality of key point heatmaps 512 and corresponding grouping tags 514. The key point heatmaps 512 depict examples of key points 30 that the machine learning model 502 identified in the example scenario. It is understood that in other example scenarios, the identified key points 30 can be different from those depicted herein. In some aspects, the machine learning model 502 also uses grouping tags, one tag for each respective key point 30. The grouping heatmaps 514 can include multiple (e.g., 5) heatmaps/channels, each heatmap representing a key point type (e.g., shaft start or shaft end). Activations with similar values (circles in 514) represent the grouping of the key points 30. In other words, key points 30 in the key point heatmaps 512 with a similar value in the grouping heatmap 514 likely belong to the same instrument 18. Model 502 learns to group key points 30 by ensuring that grouping tags associated with key points 30 in the same instrument have similar values to each other while at the same time making sure the values across different instruments are far apart.

Referring back to FIG. 5, the machine learning model 502 facilitates generating key points and grouping tags for multiple object classes, each "class" being a respective type of surgical instrument 18, such as maryland, monopolar, etc. Although the different "classes" can be broadly categorized as surgical instruments, detecting the separate classes of surgical instruments is equivalent to detecting separate types of objects/items (versus detecting surgical instruments and then categorizing them into different classes).

Further, the machine learning model 502 provides different types of key points 30 for a surgical instrument 18, for example, shaft-start 35, shaft-end 37, joint 39, tips, 31, etc. It should be noted that the machine learning model 502 facilitates key point duplicates, i.e., multiple key points 30 of the same type (e.g., tips) to be grouped and associated with a single instrument 18. Several existing machine learning models that use key points for identifying poses of humans in an image limit a single key point of each type of key point. Accordingly, aspects of the technical solutions described herein improve existing key points-based pose estimations by handling key point duplicates (e.g., multiple tips) and multiple object classes (i.e., multiple instrument types). Additionally, aspects of the technical solutions described herein facilitate providing a coarse localization of an instrument, for example, by estimating a bounding box based on the key points and corresponding grouping tags.

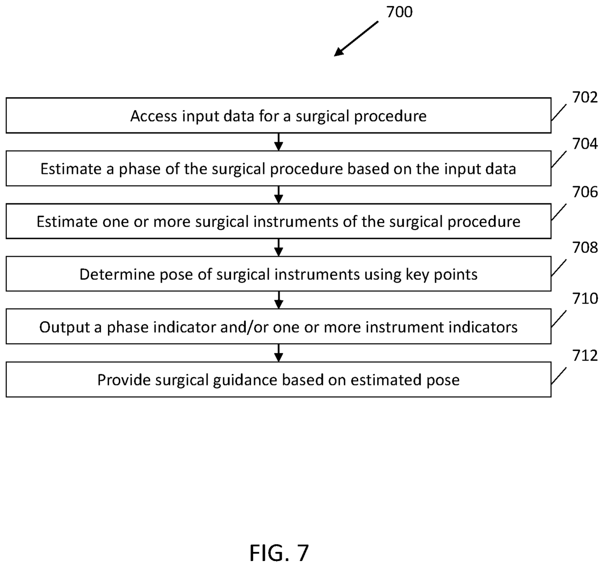

FIG. 7 depicts a flowchart of a method for detection of key points and poses of surgical instruments in surgical data using machine learning according to one or more aspects and in reference to FIGS. 1-6. Method 700 can be executed by one or more systems depicted herein as a computer-implemented method. Method 700 includes using (in an inference phase) one or more machine-learning models to detect and track poses of surgical instruments 18 using key points 30 as surgical actions are being performed in a surgical procedure.

A "pose" of an articulated surgical instrument 18 can be represented based on kinematic information using key point relative orientation. As shown in various figures herein, an articulated surgical instrument 18 is decomposed as a skeleton of individual key points. A "joint pair" can be defined as two key points which are connected within the skeleton. Based on the articulation, surgical instrument 18 is represented as a tree structure that is made up of N joints and M joint pairs. Therefore, the instrument pose estimation task is to detect the location of individual key points, and if there are multiple instruments present in the image, joints of the same instrument should be correctly associated after localization. As will be described further, to improve efficiencies, some aspects perform concurrent estimation of locations and associations between joint pairs via two branches of the same encoder-decoder predication process. In each of the blocks, features or predictions from each branch capture different structural information about the instrument and are concatenated for the next block.

At block 702, the system 100 can access input data, including, for example, video data, spatial data, and/or sensor data temporally associated with a video stream of a surgical procedure. In some aspects, at block 704, one or more machine-learning models can estimate the state of the surgical procedure based on the input data. The state of the surgical procedure can be used to automatically shortlist the type of surgical instruments 18 that may be used during that state.

At block 706, the one or more machine-learning models detect one or more surgical instruments 18 at least partially depicted in the input data. Detection of the surgical instruments 18 can include determining the key points 30 of the surgical instruments 18. The machine learning model 502 estimates the set of key points 30 for each surgical instrument 18. Further, the one or more key points 30 are grouped. The grouping is performed using the trained machine learning model (502) in one or more aspects.

As noted elsewhere herein, the set of key points for a single instrument 18 can include two or more key points 30 of the same type, e.g., two tips 31. Further, the machine learning model 502 estimates the set of key points 30 for two or more surgical instruments 18 simultaneously, and in some cases, with the two or more surgical instruments 18 being of a different type, e.g., monopolar and forceps.

At block 708, based on the key point 30, localization of one or more surgical instruments 18 is determined. The localization can include, for example, a bounding box, a medial axis, and/or any other marker identifying the location of one or more surgical instruments 18. Further, based on the identification of the type of the surgical instrument 18 and the key points 30, the machine learning model can also estimate the pose of the surgical instrument 18. A "pose" can include the position and orientation of the surgical instrument 18 in the input image. The position can be indicated by the localization. The orientation can be indicated by providing an angle of the surgical instrument with reference to a coordinate system of the input image. Alternatively, the orientation can be represented with reference to a landmark, such as an anatomical structure, another instrument 18, etc.

Upon surgical instrument detection, tracking can be performed to observe and predict the pose of the surgical instruments 18 throughout the video and with respect to other structures.

The one or more machine-learning models can include a plurality of feature encoders and task-specific decoders trained as an ensemble to detect the state and the one or more surgical instruments by sharing extracted features associated with the state and the one or more surgical instruments 18 between the feature encoders and task-specific decoders.

At block 710, one or more surgical instrument indicators temporally correlated with the video stream can be output. For example, the indicators can include icons, arrows, or other types of visual indicators that are embedded/overlaid on the video stream to indicate the estimated attributes of the surgical instruments 18. In some aspects, the indicator augments the input video. The indicator can be a graphical overlay that is rendered "above" the artifacts in the input video.

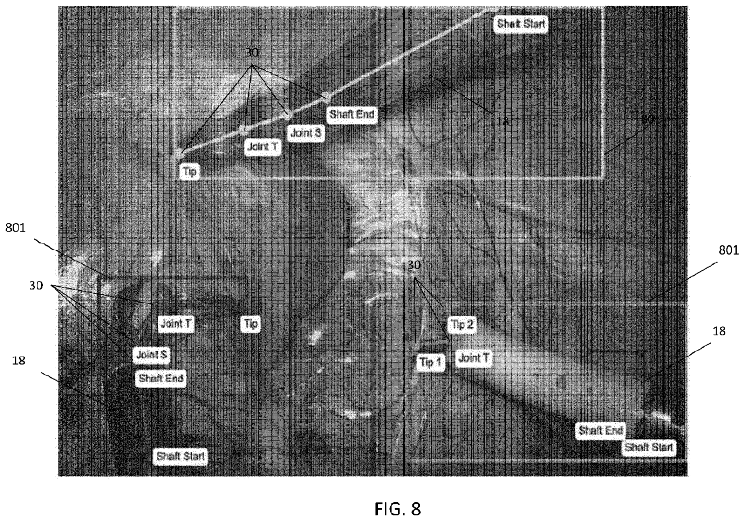

FIG. 8 depicts an example result of surgical instrument detection using one or more aspects herein. In some aspects, the indicators include markers to represent the key points 30 along with names or groups of the type of key points 30 detected. In some aspects, the key points 30 of a first instrument 18 are visually distinguished from the key points 30 of a second instrument 18. For example, visual distinction can be achieved using color, shading, pattern, border, size, icon, or any other visual attribute.

Additionally, or alternatively, the indicators can include bounding boxes 801 around the detected instruments 18. In some aspects, the bounding box 801 of a first instrument 18 is visually distinguished from a bounding box 801 of a second instrument 18. For example, visual distinction can be achieved using color, shading, pattern, border, size, icon, or any other visual attribute. In some aspects, the visual attributes of the key points 30 and the bounding box 801 associated with an instrument 18 are the same. For example, the key points 30 and bounding box 801 of a forceps are the same first color (e.g., yellow), while the key points 30 and the bounding box 801 of a monopolar are the same second color (e.g., pink). The colors used are consistent across multiple frames that are analyzed by the machine learning model(s). For example, a monopolar (or any other instrument) shown with pink (or any other color) in frame 1 is shown with the same color, i.e., pink in frame 30 (or any other frame).

FIG. 8 also depicts example results where the key points 30 that are detected are used to determine a pose of each of the surgical instruments 18 according to one or more examples. The video/image data is processed by the machine learning model 502 to identify the key points 30 and, in turn, estimate the pose of the surgical instrument 18.

Localizing surgical instruments 18 in surgical videos is an essential step toward surgical scene understanding. Instrument detection models presently available estimate a bounding box per instrument. However, due to their shape, this representation is not appropriate for surgical instruments 18 because such boxes can contain a large number of background pixels. Existing pose estimation models focus on estimating key points and grouping them into different objects. While such a detailed understanding of an instrument pose is desired, the pose estimation models struggle to learn the grouping of key points 30 in complex surgical scenes where the instruments overlap and cannot distinguish between different instrument types.

As described herein, and as shown by example results, aspects of the technical solutions described herein address the technical challenges of detecting and pose estimation of surgical instruments 18 in surgical videos. Accordingly, the technical solutions provide an improvement to computer-assisted surgery systems and to computing technology. Further, technical solutions herein provide a practical application of estimating surgical instruments automatically using machine learning models.

The technical solutions address several technical challenges with existing key point-based object detection techniques. For example, existing techniques cannot distinguish between different instrument classes, and the existing techniques are used to perform pose estimation of a single class, such as a person or a surgical instrument, without distinguishing which type of instrument it is. Aspects of technical solutions herein facilitate estimating several types of instruments (respective object classes) such as, e.g., monopolar, fenestrated, maryland, etc. Further, existing solutions fail to handle two key points of the same type (e.g., two tips 31). Instead, aspects of the technical solutions described herein address such cases.

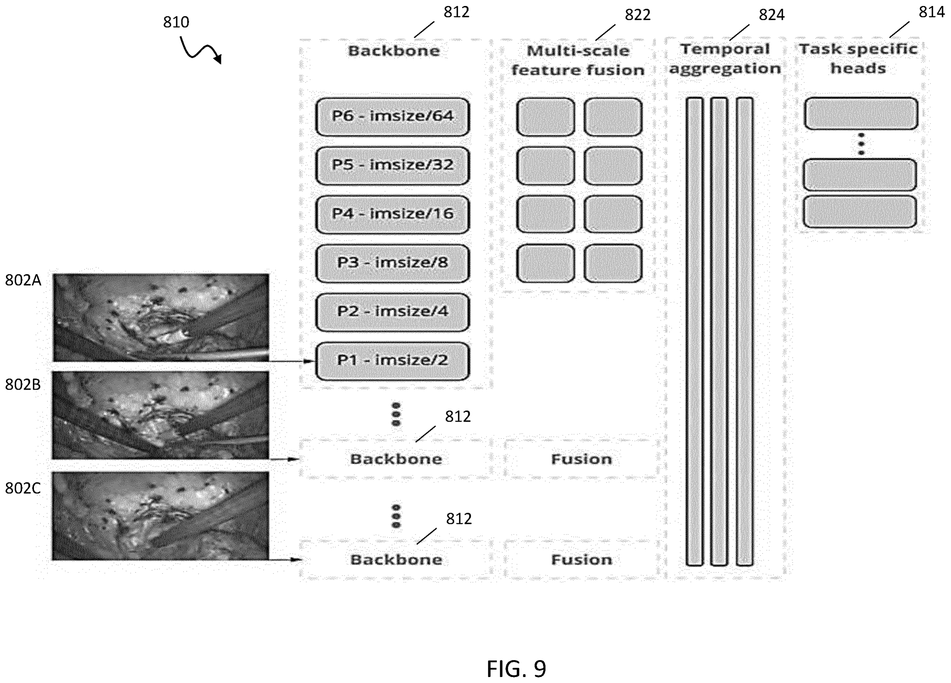

Additionally, existing techniques are restricted to performing auxiliary tasks and post-processing steps for learning the grouping of the key points. Such post-processing can have several drawbacks, including, but not limited to, being time-consuming because there is an additional post-processing step to infer the grouping of the key points from the predictions of the network. Such approaches might not be fit for real-time applications as the processing time per frame does not support real-time speeds. Only after the grouping can further operations be performed, accordingly making the existing solutions sequential and hence, time-consuming. The technical solutions described herein facilitate machine learning models (e.g., FIG. 9) that can provide real-time key point detection, key point localization, instrument detection, and instrument pose identification.

To address such technical challenges, some aspects of the technical solutions described herein jointly detect instruments (bounding box and instrument class) and estimate their pose (key points and grouping) in a unified framework. The multi-task convolutional neural network approach used by one or more aspects herein facilitates precise key point localization while simultaneously leveraging contextual spatial information to group them into individual instruments. Such direct grouping estimation improves the results compared to existing methods, where the grouping of the key points is learned through auxiliary tasks and relies on post-processing steps.

FIG. 9 depicts a machine learning model (neural network) architecture according to one or more aspects for performing joint detection and pose estimation of surgical instruments 18. The architecture 810 extends a convolutional neural network model to handle multiple instrument classes, and surgical instrument key points 30 in one or more aspects of the technical solutions described herein. For example, instrument key points 30 can include shaft-start 35, shaft-end 37, shaft joint 39, tips 31, and tip joints. It should be noted that the architecture 810 can be used to implement the machine learning model 502 in some aspects.

In one or more aspects, the machine learning model 810 is trained using a dataset composed of ~300,000 frames containing specific surgical instruments from ~200 pre-clinical and ~200 clinical videos. A split of 85/5/10 % can be used for training, validation, and test sets. It is understood that a different number of frames, instruments, or a different split can be used for training the machine learning model 810 in other aspects.

The machine learning model 810 (or neural network architecture 810) can be imagined to include two parts, a "backbone" 812, which is pre-trained (e.g., on ImageNet), and one or more "heads" 814, where each of the heads 814 can be used to perform prediction of classes, prediction of bounding boxes, key point localization, key point grouping of the surgical instruments 18.

The backbone 812 can include any type of neural networks such as VGG16, ResNet-50, SpineNet, EfficientNet-B0/B7, CSPResNeXt50, CSPDarknet53, or any other type of network. In some aspects, the neural network used to implement the backbone 812 can depend on the type of hardware (e.g., central processing unit, graphics processing unit, etc.) being used to execute the machine learning model 810.

In some aspects, multiple frames (images) of video data in the input data that is accessed (block 702) are processed concurrently. For example, as shown in FIG. 9, three frames 802A, 802B, and 802C (collectively referred to as 802) are concurrently processed by backbone 812 networks. It is understood that a different number (5, 10, 50, 100, etc.) of frames from the input video data can be processed concurrently in other aspects.

In some aspects, each input image 802 is passed through the same backbone 812. Inside the backbone 812, the spatial resolution of the features extracted from each input image 802 is gradually decreased with each convolutional block. The data is augmented before being fed further. The augmentation includes resizing the images to different sizes, rotating them, translating, modifying the colors and contrast, etc.

In some aspects, each backbone 812 processes an input image 802 at different sizes (or at different scales or resolutions). For example, the first backbone 812 processes the first input image 802A at its original image size (e.g., image size), a second image size (e.g., image size/2), and third image size (image size/4), and so on. Each different sized image is processed to identify one or more features. Feature detection can be performed using any known techniques.

The neural network architecture 810 further includes a feature-fusion component 822. The feature-fusion component 822 fuses the features detected by a backbone 812 at the different image sizes. In some aspects, each backbone 812 has a corresponding feature-fusion component for fusing the outputs of the corresponding backbone 812. Here "fusing" the features refers to combining the features from different spatial resolutions to a single-scale resolution. Depending on the type of backbone 812, the feature-fusion component 822 uses a feature pyramid network (FPN), Single Shot MultiBox Detector (SSD), or other such neural networks. The neural network architecture 810 facilitates detecting spatially variable features by fusing the multi-scaled features detected across the different sized (scaled) images.

The features that are detected and spatially fused across the different images 802 are temporally aggregated using a temporal aggregation layer 824. Aspects of the technical solutions provide an end-to-end trainable neural network architecture that does not rely on pooling and/or post-processing operations. The temporal aggregation is performed for the features detected in the several frames 802 that are concurrently analyzed.

Using the features that are detected across the spatio-temporal extent of the input data (which can be a video clip), the head(s) 814) of the neural network architecture 810 can output one or more results concurrently. For example, the multi-task neural network architecture 810 implicitly learns precise key point 30 localization while simultaneously leveraging contextual spatial information to group the key points 30 into individual instruments 18 and estimating a bounding box for each instrument 18. Accordingly, the neural network architecture 810 performs joint (i.e., combined/concurrent/simultaneous) identification and pose estimation for surgical instruments 18 in the input data (e.g., surgical video). Here, "joint" indicates that both, the identification of the surgical instruments 18 and the determination of the poses of the instruments 18 are performed as a single operation/stage. In some aspects, the result of the determination of the poses does not depend on the identification of the surgical instruments 18, and vice versa (i.e., both operations are performed independent of each other).

Accordingly, the technical solutions described herein improve technical fields, such as computing technology, surgical video analysis, computer-assisted surgical systems, etc. In addition, the technical solutions described herein provide practical applications in context-aware surgical assistance systems as they contribute to resource scheduling, surgery monitoring, decision support, etc. It is understood that the technical solutions herein provide several additional improvements and practical applications.

The technical solutions described herein are not limited to any particular type of instrument, and the pose parametrization works with any type of surgical instrument 18. Based on the pose of the surgical instrument 18, one or more examples facilitate understanding of how the surgical instrument 18 interacts with anatomy. In other examples, the pose estimation further facilitates determining and analyzing a surgical flow. For example, based on an estimated pose, a surgical action being performed can be estimated. Further yet, the estimated surgical action can be used to determine a surgical phase that is being performed.

In yet more examples, the pose estimation is used to provide real-time guidance during surgery (e.g., block 712 in FIG. 7). For example, if the pose of the surgical instrument 18 causes a particular key point 30 (e.g., tips 31) to be out of view from the video, user notification may be provided. For example, the user notification can be a warning that the tips 31 are outside the view.

Alternatively, or in addition, if the pose of the surgical instrument 18 causes a particular key point 30 (e.g., tips 31) to be within threshold proximity (e.g., predetermined proximity) of a particular anatomical structure (that is also identified by the one or more machine learning models) a user notification may be generated. For example, if an incisor is within a predetermined threshold of an artery, or any other particular anatomical structure, the user notification may be generated.

Alternatively, or in addition, if the pose of the surgical instrument 18 matches one or more threshold poses, a user notification, such as a warning, can be provided to the operator. A "threshold pose" represents a pose that may be undesirable for which a warning is provided to the operator (e.g., surgeon, medical practitioner, etc.). In one or more aspects, upon detection of such an undesirable pose, the system (100) may automatically disable one or more functionalities of the entire system (100), partial system (100), and/or the specific instrument 18 for which the threshold pose was detected. In some aspects, the "threshold pose" may be a desirable position, for example, an ideal position, to perform a surgical action.

For example, the surgeon may indicate that s/he intends to perform a particular surgical action. Alternatively, or in addition, based on the surgical state, the system 100 identifies that the particular surgical action is to be performed. System 100 identifies the threshold pose of the surgical instrument 18 for that particular surgical action. The threshold pose can be represented by specific coordinates or locations of each key point 30 of the surgical instrument 18. If the key points 30 of the surgical instrument 18 are not within a predetermined threshold of the corresponding locations specified, the surgical instrument may be considered to not match the threshold pose. If the key points 30 of the surgical instrument 18 are within the predetermined threshold of the corresponding locations specified, the surgical instrument is deemed to match the threshold pose.

In some aspects, if the surgical instrument 18 matches the threshold pose for the intended surgical action, a first indicator is provided. In some aspects, if the surgical instrument 18 does not match the threshold pose for the intended surgical action, a second indicator is provided. The first and/or second indicators can be graphical overlays that include displaying icons, animations, or other types of graphics on the video. Alternatively, or in addition, the indicators can include an audible notification. Alternatively, or in addition, the indicators can be displayed via a separate screen instead of on top of the video. It should be noted that the indicators described in this section can be used for any other indicators that are described throughout this document.

Aspects of the technical solutions described herein facilitate several technical improvements and address several technical challenges described herein. Additionally, aspects of the technical solutions described herein provide several advantages and practical applications. For example, aspects herein provide automatic real-time localization and identification of surgical instruments 18 in a video of a surgical procedure. Such localization and identification of surgical instruments 18 are used in several post-operative analytics, such as generating reports of the surgical procedure that are automatically populated to describe how one or more surgical instruments 18 were used (e.g., to perform specific surgical actions/phases). Further, the post-operative analysis of localization and identification of the surgical instruments is used for performing skill analysis, for example, to grade one or more surgeons on his/her use of the one or more surgical instruments 18.

Further, the localization and identification of the surgical instruments 18 is an important operation to estimate instrument trajectories in the surgical video or in one or more surgical phases. In some aspects, using the localization of the surgical instrument 18, one or more graphs of instrument usage are generated, for example, to depict the velocity, position, etc. of the surgical instrument 18.

For example, predefined key point 30 trajectories are compared with actual trajectories of the surgical instrument 18 to determine whether a surgeon is following certain operations when performing surgery. Based on a deviation (or matching) of the predefined and actual key point trajectories, the surgeon may be graded/trained.

The localization and identification of the surgical instrument can further facilitate automated integration tests. For example, predicted key points 30 are compared with actual user-input key point trajectories to automate daily integration tests of robotic arms.

The development of robotic surgical arms in computer-assisted surgery systems (e.g., robotic surgery systems) includes rigorous tests every time a new feature is updated/added. These tests include checks on whether the commands ordered to be performed by the robotic surgical arm (or robot) (e.g., move the instrument 18 to the left or open the instrument tips) are presently manually performed, which is tedious and time-consuming. These tests can be automated or semi-automated by using one or more aspects of the technical solutions herein to confirm the motion of the instruments 18 as commanded by the robot operator is actually performed accurately.

In one or more aspects, a surgical robotic arm can be tested automatically. The surgical robotic arm is issued a command that results in a surgical instrument 18 associated with the surgical robotic arm being in a predetermined pose. Further, a pose of the surgical instrument can be determined by one or more aspects described herein based on the one or more key points that are grouped. The pose of the surgical instrument 18 as detected and the predetermined pose are compared. If the two poses match (within a predetermined threshold), the surgical robotic arm is deemed to pass the test; otherwise, the surgical robotic arm is deemed to require maintenance. In some aspects, one or more alerts are provided to notify that the surgical robotic arm assembly may require maintenance. In some aspects, the surgical robotic arm may be prevented from being operated on until such maintenance is performed.

Further yet, in one or more aspects, the detected key points 30 are used to depict intra-operative overlays in the surgical video. The overlay can be a guide to show the surgeon 12 a direction of exit (e.g., an exit path) for the surgical instrument 18 from an existing position. For example, by identifying the shaft-start 35 to shaft-end, the surgeon 12 may identify a path to bring the tips 31 out of an anatomical structure that s/he is operating upon.

In some aspects, the output of the machine learning models is interactively annotated, for example, by the surgeon 12. Such additional data can be used to automatically generate larger datasets by updating the datasets being used to train the machine learning models described herein. Thus, the quality of the machine learning models is continuously improved by such continuous training.

The technical solutions described herein address technical challenges with existing solutions, which cannot distinguish between different classes. (e.g., different instrument types), and instead performs pose estimation only on a single class. The technical solutions herein provide improvements over existing solutions by estimating different object classes, i.e., instrument types (e.g., monopolar, fenestrated, maryland, etc.) during the joint detection and pose-estimation. Further, existing solutions cannot handle two key points of the same type (e.g., two tips). The technical solutions herein address such technical challenges for allowing detection of more than one key point of the same type. Further yet, existing solutions rely on auxiliary tasks and post-processing steps to learn the key points' groupings. Such an approach with auxiliary and post-processing has several drawbacks. The technical solutions described herein employ an architecture (see FIG. 9) to jointly learn the bounding box, key points, and the grouping of the key points. Accordingly, technical solutions herein facilitate the instrument detection in a more efficient manner.

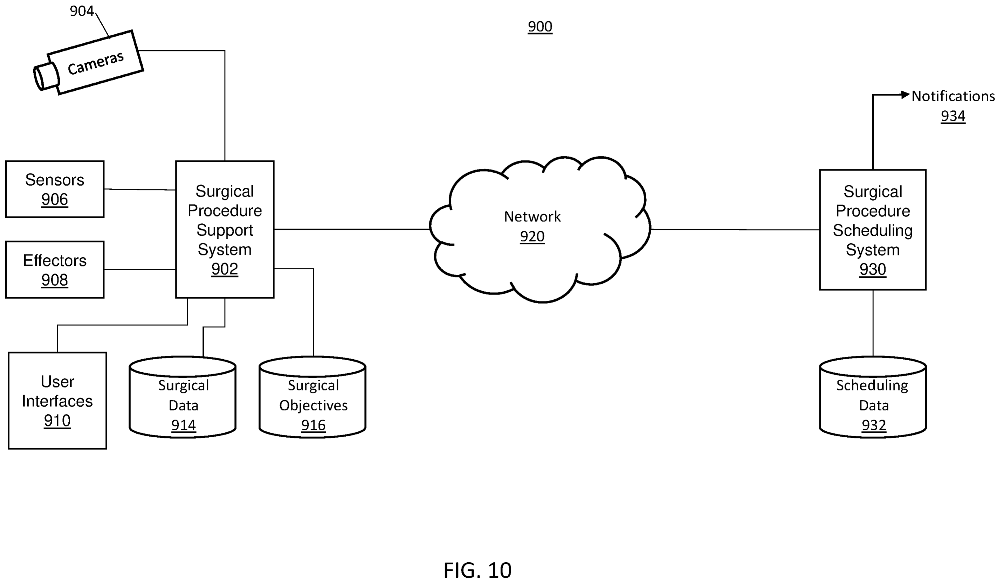

FIG. 10 depicts a surgical procedure system in accordance with one or more aspects. The example shown depicts a surgical procedure support system 902 configured to communicate with a surgical procedure scheduling system 930 through a network 920. The surgical procedure support system 902 can include or may be coupled to one or more systems described herein. The surgical procedure support system 902 can acquire image data using one or more cameras 904. The surgical procedure support system 902 can also interface with a plurality of sensors 906 and effectors 908. The sensors 906 may be associated with surgical support equipment and/or patient monitoring. The effectors 908 can be robotic components or other equipment controllable through the surgical procedure support system 902. The surgical procedure support system 902 can also interact with one or more user interfaces 910, such as various input and/or output devices. The surgical procedure support system 902 can store, access, and/or update surgical data 914 associated with a training dataset and/or live data as a surgical procedure is being performed. The surgical procedure support system 902 can store, access, and/or update surgical objectives 916 to assist in training and guidance for one or more surgical procedures.

The surgical procedure scheduling system 930 can access and/or modify scheduling data 932 used to track planned surgical procedures. The scheduling data 932 can be used to schedule physical resources and/or human resources to perform planned surgical procedures. Based on the surgical maneuver as predicted by the one or more machine learning models and a current operational time, the surgical procedure support system 902 can estimate an expected time for the end of the surgical procedure. This can be based on previously observed similarly complex cases with records in the surgical data 914. A change in a predicted end of the surgical procedure can be used to inform the surgical procedure scheduling system 930 to prepare the next patient, which may be identified in a record of the scheduling data 932. The surgical procedure support system 902 can send an alert to the surgical procedure scheduling system 930 that triggers a scheduling update associated with a later surgical procedure. The change in schedule can be captured in the scheduling data 932. Predicting an end time of the surgical procedure can increase efficiency in operating rooms that run parallel sessions, as resources can be distributed between the operating rooms. Requests to be in an operating room can be transmitted as one or more notifications 934 based on the scheduling data 932 and the predicted surgical maneuver.

As surgical maneuvers and steps are completed, progress can be tracked in the surgical data 914, and status can be displayed through the user interfaces 910. Status information may also be reported to other systems through the notifications 934 as surgical maneuvers are completed or if any issues are observed, such as complications.

The reports/views/annotations and other information described herein are added to an electronic medical record (EMR) in one or more cases. In some aspects, the information about specific surgical instruments can be stored in the patient record associated with the patient that was operated upon during the surgical procedure. Alternatively, or in addition, the information is stored in a separate database for later retrieval. The retrieval can be associated with the patient's unique identification, such as EMR-identification, social security number, or any other unique identifier. The stored data can be used to generate patient-specific reports. In some aspects, information can also be retrieved from the EMR to enhance one or more operations described herein. In one or more aspects, an operational note may be generated, which includes one or more outputs from the machine learning models. The operational note may be stored as part of the EMR.

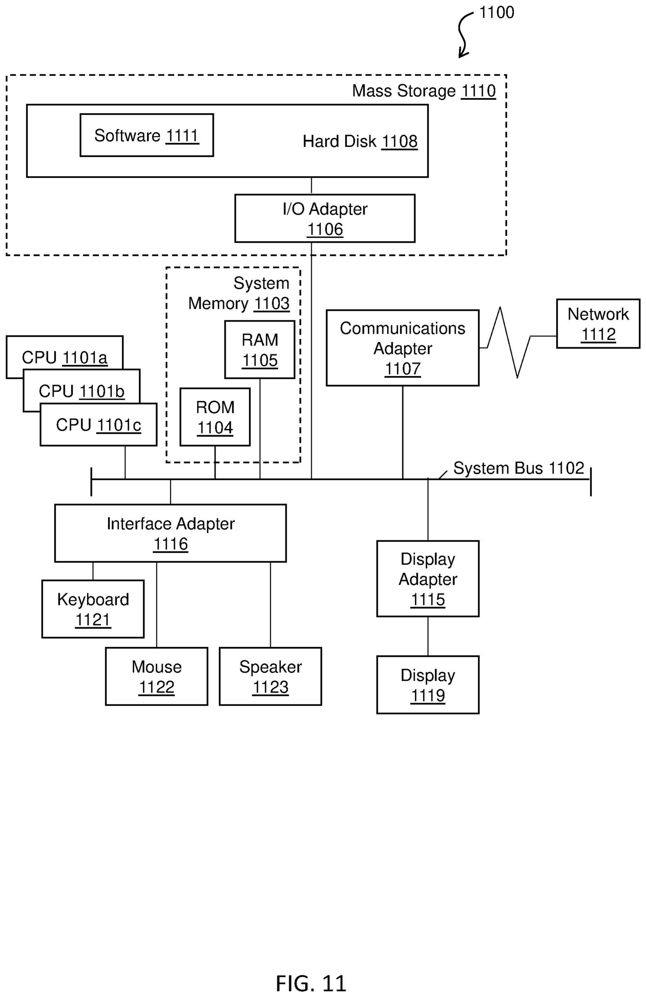

Turning now to FIG. 11, a computer system 1100 is generally shown in accordance with an embodiment. The computer system 1100 can be an electronic computer framework comprising and/or employing any number and combination of computing devices and networks utilizing various communication technologies, as described herein. The computer system 1100 can be easily scalable, extensible, and modular, with the ability to change to different services or reconfigure some features independently of others. The computer system 1100 may be, for example, a server, desktop computer, laptop computer, tablet computer, or smartphone. In some examples, computer system 1100 may be a cloud computing node. Computer system 1100 may be described in the general context of computer system executable instructions, such as program modules, being executed by a computer system. Generally, program modules may include routines, programs, objects, components, logic, data structures, and so on that perform particular tasks or implement particular abstract data types. Computer system 1100 may be practiced in distributed cloud computing environments where tasks are performed by remote processing devices that are linked through a communications network. In a distributed cloud computing environment, program modules may be located in both local and remote computer system storage media, including memory storage devices.

As shown in FIG. 11, the computer system 1100 has one or more central processing units (CPU(s)) 1101a, 1101b, 1101c, etc. (collectively or generically referred to as processor(s) 1101). The processors 1101 can be a single-core processor, multi-core processor, computing cluster, or any number of other configurations. The processors 1101, also referred to as processing circuits, are coupled via a system bus 1102 to a system memory 1103 and various other components. The system memory 1103 can include a read only memory (ROM) 1104 and a random access memory (RAM) 1105. The ROM 1104 is coupled to the system bus 1102 and may include a basic input/output system (BIOS), which controls certain basic functions of the computer system 1100. The RAM is read-write memory coupled to the system bus 1102 for use by the processors 1101. The system memory 1103 provides temporary memory space for operations of said instructions during operation. The system memory 1103 can include random access memory (RAM), read only memory, flash memory, or any other suitable memory systems.

The computer system 1100 comprises an input/output (I/O) adapter 1106 and a communications adapter 1107 coupled to the system bus 1102. The I/O adapter 1106 may be a small computer system interface (SCSI) adapter that communicates with a hard disk 1108 and/or any other similar component. The I/O adapter 1106 and the hard disk 1108 are collectively referred to herein as a mass storage 1110.

Software 1111 for execution on the computer system 1100 may be stored in the mass storage 1110. The mass storage 1110 is an example of a tangible storage medium readable by the processors 1101, where the software 1111 is stored as instructions for execution by the processors 1101 to cause the computer system 1100 to operate, such as is described herein below with respect to the various Figures. Examples of computer program product and the execution of such instruction is discussed herein in more detail. The communications adapter 1107 interconnects the system bus 1102 with a network 1112, which may be an outside network, enabling the computer system 1100 to communicate with other such systems. In one embodiment, a portion of the system memory 1103 and the mass storage 1110 collectively store an operating system, which may be any appropriate operating system to coordinate the functions of the various components shown in FIG. 11.

Additional input/output devices are shown as connected to the system bus 1102 via a display adapter 1115 and an interface adapter 1116. In one embodiment, the adapters 1106, 1107, 1115, and 1116 may be connected to one or more I/O buses that are connected to the system bus 1102 via an intermediate bus bridge (not shown). A display 1119 (e.g., a screen or a display monitor) is connected to the system bus 1102 by a display adapter 1115, which may include a graphics controller to improve the performance of graphics intensive applications and a video controller. A keyboard 1121, a mouse 1122, a speaker 1123, etc., can be interconnected to the system bus 1102 via the interface adapter 1116, which may include, for example, a Super I/O chip integrating multiple device adapters into a single integrated circuit. Suitable I/O buses for connecting peripheral devices such as hard disk controllers, network adapters, and graphics adapters typically include common protocols, such as the Peripheral Component Interconnect (PCI). Thus, as configured in FIG. 11, the computer system 1100 includes processing capability in the form of the processors 1101, and, storage capability including the system memory 1103 and the mass storage 1110, input means such as the keyboard 1121 and the mouse 1122, and output capability including the speaker 1123 and the display 1119.

In some embodiments, the communications adapter 1107 can transmit data using any suitable interface or protocol, such as the internet small computer system interface, among others. The network 1112 may be a cellular network, a radio network, a wide area network (WAN), a local area network (LAN), or the Internet, among others. An external computing device may connect to the computer system 1100 through the network 1112. In some examples, an external computing device may be an external webserver or a cloud computing node.

It is to be understood that the block diagram of FIG. 11 is not intended to indicate that the computer system 1100 is to include all of the components shown in FIG. 11. Rather, the computer system 1100 can include any appropriate fewer or additional components not illustrated in FIG. 11 (e.g., additional memory components, embedded controllers, modules, additional network interfaces, etc.). Further, the embodiments described herein with respect to computer system 1100 may be implemented with any appropriate logic, wherein the logic, as referred to herein, can include any suitable hardware (e.g., a processor, an embedded controller, or an application specific integrated circuit, among others), software (e.g., an application, among others), firmware, or any suitable combination of hardware, software, and firmware, in various embodiments.

The examples described herein can be performed using a computer such as a server computer, a desktop computer, a tablet computer, etc. In one or more examples, the technical solutions herein can be implemented using cloud computing technology.

The present invention may be a system, a method, and/or a computer program product at any possible technical detail level of integration. The computer program product may include a computer-readable storage medium (or media) having computer-readable program instructions thereon for causing a processor to carry out aspects of the present invention.

The computer-readable storage medium can be a tangible device that can retain and store instructions for use by an instruction execution device. The computer-readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer-readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer-readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer-readable program instructions described herein can be downloaded to respective computing/processing devices from a computer-readable storage medium or to an external computer or external storage device via a network, for example, the Internet, a local area network, a wide area network, and/or a wireless network. The network may comprise copper transmission cables, optical transmission fibers, wireless transmission, routers, firewalls, switches, gateway computers, and/or edge servers. A network adapter card or network interface in each computing/processing device receives computer-readable program instructions from the network and forwards the computer-readable program instructions for storage in a computer-readable storage medium within the respective computing/processing device.

Computer-readable program instructions for carrying out operations of the present invention may be assembler instructions, instruction-set-architecture (ISA) instructions, machine instructions, machine-dependent instructions, microcode, firmware instructions, state-setting data, configuration data for integrated circuitry, or either source-code or object code written in any combination of one or more programming languages, including an object-oriented programming language such as Smalltalk, C++, or the like, and procedural programming languages, such as the "C" programming language or similar programming languages. The computer-readable program instructions may execute entirely on the user's computer, partly on the user's computer, as a stand-alone software package, partly on the user's computer and partly on a remote computer, or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some aspects, electronic circuitry, including, for example, programmable logic circuitry, field-programmable gate arrays (FPGA), or programmable logic arrays (PLA), may execute the computer-readable program instruction by utilizing state information of the computer-readable program instructions to personalize the electronic circuitry, in order to perform aspects of the present invention.

Aspects of the present invention are described herein with reference to flowchart illustrations and/or block diagrams of methods, apparatus (systems), and computer program products according to aspects of the invention. It will be understood that each block of the flowchart illustrations and/or block diagrams and combinations of blocks in the flowchart illustrations and/or block diagrams can be implemented by computer-readable program instructions.

These computer-readable program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer-readable program instructions may also be stored in a computer-readable storage medium that can direct a computer, a programmable data processing apparatus, and/or other devices to function in a particular manner, such that the computer-readable storage medium having instructions stored therein comprises an article of manufacture including instructions which implement aspects of the function/act specified in the flowchart and/or block diagram block or blocks.

The computer-readable program instructions may also be loaded onto a computer, other programmable data processing apparatus, or other device to cause a series of operational steps to be performed on the computer, other programmable apparatus, or other devices to produce a computer-implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various aspects of the present invention. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

The descriptions of the various aspects of the present invention have been presented for purposes of illustration but are not intended to be exhaustive or limited to the aspects disclosed. Many modifications and variations will be apparent to those of ordinary skill in the art without departing from the scope and spirit of the described aspects. The terminology used herein was chosen to best explain the principles of the aspects, the practical application or technical improvement over technologies found in the marketplace, or to enable others of ordinary skill in the art to understand the aspects described herein.

Various aspects of the invention are described herein with reference to the related drawings. Alternative aspects of the invention can be devised without departing from the scope of this invention. Various connections and positional relationships (e.g., over, below, adjacent, etc.) are set forth between elements in the following description and in the drawings. These connections and/or positional relationships, unless specified otherwise, can be direct or indirect, and the present invention is not intended to be limiting in this respect. Accordingly, a coupling of entities can refer to either a direct or an indirect coupling, and a positional relationship between entities can be a direct or indirect positional relationship. Moreover, the various tasks and process steps described herein can be incorporated into a more comprehensive procedure or process having additional steps or functionality not described in detail herein.

The following definitions and abbreviations are to be used for the interpretation of the claims and the specification. As used herein, the terms "comprises," "comprising," "includes," "including," "has," "having," "contains," or "containing," or any other variation thereof is intended to cover a non-exclusive inclusion. For example, a composition, a mixture, process, method, article, or apparatus that comprises a list of elements is not necessarily limited to only those elements but can include other elements not expressly listed or inherent to such composition, mixture, process, method, article, or apparatus.

Additionally, the term "exemplary" is used herein to mean "serving as an example, instance or illustration." Any aspect or design described herein as "exemplary" is not necessarily to be construed as preferred or advantageous over other aspects or designs. The terms "at least one" and "one or more" may be understood to include any integer number greater than or equal to one, i.e., one, two, three, four, etc. The term "a plurality" may be understood to include any integer number greater than or equal to two, i.e., two, three, four, five, etc. The term "connection" may include both an indirect "connection" and a direct "connection."

The terms "about," "substantially," "approximately," and variations thereof are intended to include the degree of error associated with the measurement of the particular quantity based upon the equipment available at the time of filing the application. For example, "about" can include a range of ± 8% or 5%, or 2% of a given value.

For the sake of brevity, conventional techniques related to making and using aspects of the invention may or may not be described in detail herein. In particular, various aspects of computing systems and specific computer programs to implement the various technical features described herein are well known. Accordingly, in the interest of brevity, many conventional implementation details are only mentioned briefly herein or are omitted entirely without providing the well-known system and/or process details.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), and field programmable logic arrays (FPGAs), or other equivalent integrated or discrete logic circuitry. Accordingly, the term "processor" as used herein may refer to any of the foregoing structures or any other physical structure suitable for implementation of the described techniques. Also, the techniques could be fully implemented in one or more circuits or logic elements.

### Numbered Statements of Invention

1. A system comprising:
   a memory device; and
   one or more processors coupled with the memory device, the one or more processors configured to:
   identify, autonomously, one or more key points associated with a plurality of surgical instruments in a video of a surgical procedure;
   group the one or more key points according to the plurality of surgical instruments using a first machine learning model; and
   determine, based on the one or more key points that are grouped, poses of the surgical instruments and types of the surgical instruments respectively using a second machine learning model, wherein the poses and the types are determined concurrently.
2. The system of statement 1, wherein a pose of a surgical instrument from the plurality of surgical instruments is used to provide user feedback.
3. The system of statement 1, wherein the one or more processors are further configured to generate a bounding box of a surgical instrument based on the one or more key points grouped according to the surgical instrument.
4. The system of statement 1, wherein the video of the surgical procedure is captured by an endoscopic camera from inside a patient's body.
5. The system of statement 1, wherein the video of the surgical procedure is captured by a camera from outside a patient's body.
6. The system of statement 1, wherein the first machine learning model outputs an annotation for each of the one or more key points identified.
7. The system of statement 1, wherein the poses and types of the surgical instruments are identified with temporal continuity.
8. The system of statement 1, wherein the one or more processors are configured to test a surgical robotic arm, the test comprising:
   issuing a command to the surgical robotic arm that results in a surgical instrument associated with the surgical robotic arm to be in a predetermined pose;
   determining a first pose of the surgical instrument based on the one or more key points that are grouped; and
   comparing the first pose and the predetermined pose.
9. A computer-implemented method comprising:
   identifying, autonomously, one or more key points associated with a plurality of surgical instruments in a video of a surgical procedure;
   grouping a set of key points from the one or more key points associated with a surgical instrument using a first machine learning model; and
   determining, based on the set of key points that are grouped, a pose of the surgical instrument.
10. The computer-implemented method of statement 9, further comprising depicting a graphical overlay on the video to indicate the identified pose of the surgical instrument.
11. The computer-implemented method of statement 10, wherein the graphical overlay includes a depiction of the one or more key points to identify an exit path to move the surgical instrument.
12. The computer-implemented method of statement 9, further comprising in response to the pose of the surgical instrument matching a threshold pose, generating a user notification.
13. The computer-implemented method of statement 12, wherein the user notification is a first user notification, and in response to the pose of the surgical instrument not matching the threshold pose, generating a second user notification, different from the first user notification.
14. The computer-implemented method of statement 12, wherein the threshold pose is indicative of a desired pose of the surgical instrument based on a surgical action to be performed.
15. The computer-implemented method of statement 12, wherein the threshold pose is indicative of an undesired pose of the surgical instrument.
16. The computer-implemented method of statement 12, wherein the user notification includes an audible notification.
17. The computer-implemented method of statement 12, wherein the user notification is provided on a separate display, distinct from the video.
18. A computer program product comprising a memory device with computer-readable instructions stored thereon, wherein executing the computer-readable instructions by one or more processing units causes the one or more processing units to perform a method comprising:
   accessing, a video of a surgical procedure comprising use of a plurality of surgical instruments concurrently;
   identifying, autonomously, one or more key points associated with the surgical instruments;
   concurrently performing, using one or more machine learning models:
      grouping a set of key points from the one or more key points, the set of key points associated with a surgical instrument;
      identifying a type of the surgical instrument based on the set of key points; and
      estimating a pose of the surgical instrument based on the set of key points; and
   augmenting the video of the surgical procedure in response to a key point of the surgical instrument being out of view from the video.
19. The computer program product of statement 18, wherein the one or more machine learning models comprise multi-tasking convolutional neural network layers that aggregate spatio-temporal features in one or more frames of the video.
20. The computer program product of statement 18, wherein the method further comprises augmenting the video of the surgical procedure in response to the key point of the surgical instrument being within a predetermined proximity of an anatomical structure.

## Claims

1. A computer program product comprising a memory device with computer-readable instructions stored thereon, wherein executing the computer-readable instructions by one or more processing units causes the one or more processing units to perform a method comprising:
accessing, a video of a surgical procedure comprising use of a plurality of surgical instruments concurrently;
identifying, autonomously, one or more key points associated with the surgical instruments;
concurrently performing, using one or more machine learning models:
grouping a set of key points from the one or more key points, the set of key points associated with a surgical instrument;
identifying a type of the surgical instrument based on the set of key points; and
estimating a pose of the surgical instrument based on the set of key points; and
augmenting the video of the surgical procedure in response to a key point of the surgical instrument being out of view from the video.

2. The computer program product of claim 1, wherein the one or more machine learning models comprise multi-tasking convolutional neural network layers that aggregate spatiotemporal features in one or more frames of the video.

3. The computer program product of either of claims 1 or 2, wherein the method further comprises:
augmenting the video of the surgical procedure in response to the key point of the surgical instrument being within a predetermined proximity of an anatomical structure.

4. The computer program product of any of claims 1 to 3, wherein the pose and types of the surgical instruments are identified with temporal continuity.

5. The computer program product of any of claims 1 to 4, wherein the video of the surgical procedure is captured by an endoscopic camera from inside a patient's body or a camera from outside a patient's body.

6. A computer implemented method comprising:
accessing, a video of a surgical procedure comprising use of a plurality of surgical instruments concurrently;
identifying, autonomously, one or more key points associated with the surgical instruments;
concurrently performing, using one or more machine learning models:
grouping a set of key points from the one or more key points, the set of key points associated with a surgical instrument;
identifying a type of the surgical instrument based on the set of key points; and
estimating a pose of the surgical instrument based on the set of key points; and
augmenting the video of the surgical procedure in response to the key point of the surgical instrument being within a predetermined proximity of an anatomical structure.

7. The computer implemented method of claim 6, wherein the one or more machine learning models comprise multi-tasking convolutional neural network layers that aggregate spatiotemporal features in one or more frames of the video.

8. The computer implemented method of either of claims 6 or 7, wherein the method further comprises:
augmenting the video of the surgical procedure in response to a key point of the surgical instrument being out of view from the video.

9. The computer implemented method of any of claims 6 to 8, wherein the pose and types of the surgical instruments are identified with temporal continuity.

10. The computer implemented method of any of claims 6 to 9, wherein the video of the surgical procedure is captured by an endoscopic camera from inside a patient's body or a camera from outside a patient's body.

11. A system comprising:
a memory device; and
one or more processors coupled with the memory device, the one or more processors configured to:
access a video of a surgical procedure comprising use of a plurality of surgical instruments concurrently;
identify, autonomously, one or more key points associated with the surgical instruments;
concurrently perform, using one or more machine learning models:
grouping a set of key points from the one or more key points, the set of key points associated with a surgical instrument;
identifying a type of the surgical instrument based on the set of key points; and
estimating a pose of the surgical instrument based on the set of key points; and
augment the video of the surgical procedure in response to a key point of the surgical instrument being out of view from the video.

12. The system of claim 11, wherein the one or more machine learning models comprise multi-tasking convolutional neural network layers that aggregate spatiotemporal features in one or more frames of the video.

13. The system of either of claims 11 or 12, wherein the method further comprises:
augmenting the video of the surgical procedure in response to the key point of the surgical instrument being within a predetermined proximity of an anatomical structure.

14. The system of any of claims 11 to 13, wherein the pose and types of the surgical instruments are identified with temporal continuity.

15. The system of any of claims 11 to 14, wherein the video of the surgical procedure is captured by an endoscopic camera from inside a patient's body or a camera from outside a patient's body.
